# EUROPÄISCHE PATENTANMELDUNG

(11) **EP 2 229 881 A1**
(43) Veröffentlichungstag der Anmeldung: **22.09.2010**
(21) Anmeldenummer: 10000441.5
(22) Anmeldetag: 19.01.2010
(51) Int. Cl.: A61B 5/0408, A61N 1/04, A61B 5/0424

(54) **Biomedizinische Elektrode**

(30) Priorität: 19.03.2009 DE 102009013470
(71) Anmelder: Covidien AG, 8212 Neuhausen am Rheinfall (CH)
(72) Erfinder: Malkowsky, Andrea, 38828 Wegeleben (DE); Anz, Johannes, 38828 Wegeleben (DE)
(74) Vertreter: Olsson, Carl H.S.

(57) **Zusammenfassung**

Die Erfindung bezieht sich auf eine biomedizinische Elektrode, als nichtinvasive, selbstklebende, flexible Liquidgelelektrode zur elektrischen Signalübertragung von der Haut oder zur Haut bei Menschen und Tieren, wobei die Elektrode zum einmaligen Gebrauch bestimmt ist.

Ausgehend von den Nachteilen des bekannten Standes der Technik soll eine biomedizinische Elektrode mit einem Liquidgel geschaffen werden, die ohne nachteilige Auswirkungen auf die Signalübertragung eine ausreichende und hautschonende Haftung auf der Haut gewährleistet und sich durch einen kostengünstigen Aufbau auszeichnet. Hierzu wird als Lösung vorgeschlagen, dass zwischen dem ablösbaren Liner 8 und der zur Haut zeigenden Seite des Gelkissens 6 eine dünnwandige, flexible, flüssigkeitsdurchlässige, nassfeste Abdeckung 10, 10a angeordnet ist. Die ist so dimensioniert, dass die in Richtung zur Haut zeigende Fläche des Gelkissens 6 vollständig überdeckt wird und einen umlaufenden Befestigungsrand 10a aufweist. Dieser ist an der zur Haut zeigenden Seite des Trägermaterials 1 oder Labels 3 dauerhaft fest mit diesem verbunden. Die zur Haut zeigende Fläche der Abdeckung 10 steht während der Applikation der Elektrode mit der Haut in Berührungskontakt, wobei Liquidgel 7 durch die Abdeckung 10 hindurch an den vom Gelkissen 6 überdeckten Bereich der Hautoberfläche gelangt.

## Beschreibung

Die Erfindung bezieht sich auf eine biomedizinische Elektrode, als nichtinvasive, selbstklebende, flexible Liquidgelelektrode zur elektrischen Signalübertragung von der Haut oder zur Haut bei Menschen und Tieren, wobei die Elektrode zum einmaligen Gebrauch bestimmt ist.

Derartige Einmalelektroden werden für unterschiedliche klinische Anwendungen, wie z.B. Elektrokardiographie (EKG), Elektroenzephalographie (EEG), Elektromyogramm (EMG), Oberflächenelektrodenmyographie (OEMG), Elektrookulographie (EOG), Impedanzkardiographie (IKG), Muskelstimulation, Elektro- Impedanz- Tomographie (EIT), eingesetzt.

Die Elektroden können zur Übertragung der elektrischen Signale grundsätzlich mit einem Solidgel oder Liquidgel ausgerüstet sein.

Im Unterschied zu Solidgelektroden, mit einem durch Polymerisation oder Vernetzung verfestigtem Gelkörper, besitzen Liquidgelelektroden einen saugfähigen Aufnahmekörper (Gelkissen), der beispielsweise aus einem offenzelligen Polymerschaum oder einem Textilgebilde besteht und mit einer ionisch leitfähigen, mindestens gegen ihr Ausfließen unter normaler Schwerkrafteinwirkung angedickten Flüssigkeit (dem sogenannten Liquidgel) getränkt ist. Über das Liquidgel, das nach der Applikation der Elektrode in direktem Kontakt mit der Haut steht, erfolgt die Signalübertragung an einen elektrisch leitenden Sensor, der mit der Anschlussleitung eines peripheren Gerätes verbindbar ist.

Als geeignete ionisch leitende Flüssigkeiten kommen insbesondere wässrige Lösungen von Halogensalzen der Erdalkalimetalle, wie Natrium- oder Kaliumchlorid, zur Anwendung. Medizinische Einweg-Elektroden auf Liquidgelbasis unter Verwendung verschiedener, meist selbstklebend ausgerüsteter, flexibler Trägermaterialien, wie Polymerschaum, Vliesen und Geweben, sind bereits seit langem bekannt (z.B. US 3 834 373 A, US 3 989 035 A und DE 24 42 142 A). Im Vergleich zu Solidgelelektroden erzeugen Liquidgelelektroden üblicherweise eine bessere Signalqualität, vor allem verursacht durch eine in Größenordnungen niedrigere Hautimpedanz.

Aus der US 6 650 922 B2 ist eine Einmalelektrode bekannt, bei der das elektrische Anschlusselement mit einem schwammartigen Körper (Gelkissen) in Verbindung steht, der mit einem Liquidgel getränkt ist. Das Gelkissen ist von einer schützenden lösbaren Abdeckung umgeben, die vor der Applikation der Elektrode entfernt werden muss. Die Abdeckung soll dafür sorgen, dass Liquidgel und Klebstoffbeschichtung während der Lagerung der Elektroden nicht austrocknen bzw. permanent klebende Oberflächen nicht zu ungewollten Hafteffekten vor der eigentlichen Anwendung führen. Das Anschlusselement ist mittels einer auf der Oberseite des Trägermaterials aufklebbaren Folie befestigt. Nach Entfernung der Abdeckung wird die Elektrode mit den klebfähigen Abschnitten des Trägermaterials auf der Haut befestigt, wobei die ionisch leitende Flüssigkeit des Gelkissens mit der Haut in Kontakt steht.

Eine in ihrem Aufbau ähnliche Elektrode ist auch in der DE 299 15 267 U1 beschrieben.

Die Gelkissen können aus unterschiedlichen Materialien bestehen, besonders gut geeignet ist ein retikulierter (offenzelliger) Polyurethanschaum. Derartige Gelkissen mit dauerelastischem Verhalten haben ein gutes Absorptionsvermögen für liquide Gele. Ihr Nachteil besteht darin, dass ihre offenen, scharfen Zellkanten bei der praktischen Anwendung auf empfindlicher Haut mechanische Reizungen verursachen können, die sich in Rötung der betreffenden Stelle und unangenehmem Juckreiz äußern können.

Ein häufig auftretendes Problem bei der Anwendung von Liquidgelelektroden ist, dass das Liquidgel unter äußerer, mechanischer Einwirkung, wie beim Anschließen des Kabeladapters oder der Bewegung eines Patienten, oder allein infolge der Kapillarwirkung der Grenzfläche zwischen Haut und Haftklebstoff des Trägermaterials die Neigung hat, eine antiadhäsive Trennschicht zwischen der Haut und dem Haftklebstoff des Trägermaterials der Elektrode zu bilden. Dieser Trennfilm weitet sich mit zunehmender Applikationszeit immer weiter aus, so dass die Elektrode zumindest partiell den Hautkontakt verliert oder gar völlig abfällt. Die Folge sind Signalstörungen bis hin zu -unterbrechungen.

Der Erfindung liegt die Aufgabe zugrunde, eine biomedizinische Elektrode mit einem Liquidgel zu schaffen, die ohne nachteilige Auswirkungen auf die Signalübertragung eine ausreichende und hautschonende Haftung auf der Haut gewährleistet und sich durch einen kostengünstigen Aufbau auszeichnet.

Erfindungsgemäß wird die Aufgabe durch die im Anspruch 1 angegebenen Merkmale gelöst. Vorteilhafte Ausgestaltungen und Weiterbildungen sind Gegenstand der Ansprüche 2 bis 9. Zwischen dem ablösbaren Liner und der zur Haut zeigenden Seite des Gelkissens der Elektrode ist eine dünnwandige, flexible, flüssigkeitsdurchlässige, nassfeste Abdeckung angeordnet. Diese ist so dimensioniert, dass die in Richtung zur Haut zeigende Fläche des Gelkissens vollständig überdeckt wird. Der umlaufende Befestigungsrand der Abdeckung ist an der zur Haut zeigenden Seite des Trägermaterials dauerhaft fest mit diesem verbunden. Die Anordnung einer zusätzlichen vliesartigen Abdeckung für das Gelkissen verhindert überraschenderweise, dass bei mechanischen Beanspruchungen der applizierten Elektrode Liquidgel in den Bereich der hautseitigen Befestigung der Elektrode gelangen kann. In Versuchen zeigte sich, dass die Liquidgelelektrode auch nach einer mehrstündigen Tragedauer und unter relativ intensiven Bewegungen des Probanden noch fest auf der Haut haften blieb. Die Abdeckung besteht vorzugsweise aus einem vliesartigen Material mit einem Flächengewicht von 5 bis 80, vorzugsweise 15 bis 30 g/m². Als Materialien sind beispielsweise Polyester- oder Polyethylen-Fasern gut geeignet. Prinzipiell kann die Abdeckung auch aus anderen gewebten oder ungewebten Textil- oder Polymermaterialien, wie z.B. flexible, biokompatible, alterungsbeständige Materialen auf Basis von PE, PUR oder PP in einer Dicke von 0,01 bis 0,25 mm, bestehen, die die geforderten Eigenschaften aufweisen.

Um die erforderliche Durchlassfähigkeit für das Liquidgel zu gewährleisten, muss die Abdeckung kleine Öffnungen bzw. Poren aufweisen, die es ermöglichen, dass auf der hautzugewandten Seite ein gleichmäßiger, die Haut benetzender dünner Gelfilm gebildet werden kann, über den der elektrische Kontakt hergestellt wird. Die Öffnungen bzw. Perforationen sollten im Abstand von 0,005 bis 3 mm, vorzugsweise 0,5 bis 1,5 mm angeordnet sein. Diese können durch An- oder Ausstanzen erzeugt werden, wobei die Ausdehnung zwischen 0,2 und 2 mm bzw. der Durchmesser der Öffnungen zwischen 0,1 und 1 mm betragen sollte. Textile Materialien besitzen per se eine porenartige Struktur. Außerdem sollte die Abdeckung aus einem Material bestehen, das hautverträglich ist und auch bei empfindlicher Haut zu keinen Hautreizungen führt. Insbesondere die Fläche der Abdeckung, die mit der Haut in Kontakt gelangt, sollte eine samtweiche Oberfläche bzw. Struktur besitzen.

Die äußere Form der Abdeckung richtet sich nach der jeweiligen Geometrie des Gelkissens. Die Elektrode kann so ausgeführt sein, dass das Trägermaterial eine Öffnung besitzt, in die das Gelkissen eingesetzt ist. Der überstehende, umlaufende Rand der Abdeckung ist an dem Trägermaterial mittels einer Klebstoffschicht flüssigkeitsdicht befestigt. Hierfür kann ein mittels unter Druck und Wärme schmelzbarer Klebstoff eingesetzt werden.

Auf der hautabgewandten Seite ist ein Label angeordnet, das das Gelkissen vollständig überdeckt und flüssigkeitsdicht mit dem Trägermaterial verbunden ist. Die zur Haut zeigende Seite des Trägermaterials ist vollflächig mit einem ablösbaren Liner verbunden, der eine Kavität besitzt, die den überstehenden Teil des Gelkissens aufnimmt.

Gemäß einer anderen Elektrodenausführung ist das Gelkissen auf der zur Haut zeigenden Seite eines flüssigkeitsdichten, vollflächigen Trägermaterials angeordnet. Der überstehende, umlaufende Rand der Abdeckung ist an dem Trägermaterial mittels einer Klebstoffschicht flüssigkeitsdicht befestigt. Die zur Haut zeigende Seite des Trägermaterials ist vollflächig mit einem ablösbaren Liner verbunden, der eine Kavität besitzt, die den überstehenden Teil des Gelkissens aufnimmt.

Die aus Metalldruckknopf und dem Sensor bestehende elektrische Verbindung ist entweder in dem Label oder in dem Trägermaterial fixiert.

Die Tränkung des Gelkissens mit Liquidgel sollte erst unmittelbar vor dem Aufsetzen des Liners erfolgen.

Das Gelkissen besteht aus einem flexiblen, naßfesten und saugfähigen Material, das in Verbindung mit dem Liquidgel und den übrigen Komponenten keinerlei störende, chemische Wechselwirkungen eingeht. Das Material für das Gelkissen kann aus einem gewebten oder ungewebten textilen Gebilde oder einem mindestens teilweise offenzelligen Schaum oder einer Kombinationen beider bestehen. Bevorzugt wird das Gelkissen aus retikuliertem Polyurethanschaum hergestellt.

In Abhängigkeit von der Elektrodengröße kommen Gelkissen mit einer Fläche zwischen 3 und 1000 mm², vorzugsweise zwischen 100 und 500 mm², zum Einsatz.

Die Höhe der Gelkissens beträgt 0,1 bis 7 mm, vorzugsweise 1,5 bis 3 mm, wobei dieses jedoch im Regelfall mindesten so dick sein sollte wie das Trägermaterial.

Das Trägermaterial kann z.B. aus einem flexiblen Material auf Basis eines Polymerschaums, einer kompakten Polymerfolie, eines Vlieses oder Gewebes bestehen und ist mindestens hautseitig mit einem hautverträglichen Permanentklebstoff ausgerüstet. Die Sensorkombination besteht aus einem gelverträglichen, mindestens oberflächlich elektrisch leitenden Körper, vorzugsweise mit einer Silber- Silberchlorid- Oberfläche. Die weitere elektrische Verbindung zu einem peripheren Gerät erfolgt mittels üblicher Leitungen, die fix oder über entsprechende Adapter lösbar mit dem Sensor verbunden sind.

Die Kombination Druckknopf/Sensor ist analog ausgebildet wie bei handelsüblichen EKG-Elektroden. Diese kann auch röntgentransluzent ausgeführt sein. Zum Entfernen des Liners unmittelbar vor der Applikation der Elektroden ist ein Teil des Trägermaterials als nichtklebende Grifflasche ausgebildet.

Unmittelbar vor Applikation der Elektrode kann auf die Haut eine für EKG-Elektroden übliche, abrasiv und befeuchtend wirkende Creme aufgetragen werden, um die Hautimpedanz abzusenken. Somit können rauscharme Ableitsignale höchster Qualität erzielt werden.

Die Erfindung soll nachstehend an einem Beispiel erläutert werden. In der zugehörigen Zeichnung zeigen:
- Fig. 1: die Draufsicht auf eine erste Ausführung der erfindungsgemäßen Elektrode,
- Fig. 2: die Elektrode gemäß Fig. 1 als Explosionsdarstellung und
- Fig. 3: eine zweite Ausführung der erfindungsgemäßen Elektrode als Explosionsdarstellung.

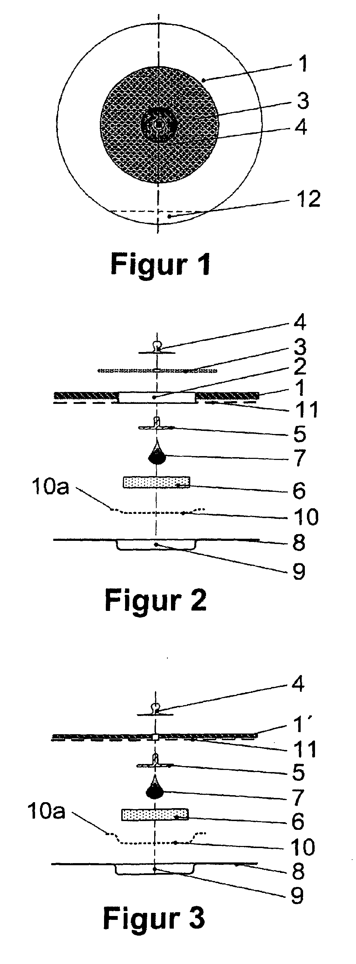

Die in den Figuren 1 und 2 gezeigte Elektrode besteht aus einer mit hautverträglichem Permanentklebstoff beschichteten Polyethylen-Schaumfolie 1 als Trägermaterial. Die Materialdicke beträgt ca. 1 mm. Die Klebstoffschicht 11 befindet sich auf der hautzugewandten Oberfläche des Trägermaterials 1. Das kreisrunde Trägermaterial 1 weist mittig eine Öffnung 2 auf, in die das mit einem ionisch leitenden Gel 7 getränkte Gelkissen 6 eingesetzt ist. Das Gelkissen 6 besteht aus einem offenzelligen Polyurethanschaum. In dieses ist eine wässrige Natriumchloridlösung mit üblichen Zusätzen wie Konservierungsstoffen, Farbzusätzen und Feuchthaltemitteln als ionisch leitende Flüssigkeit injiziert, die rheologisch so eingestellt wurde, dass sie unter normalen Schwerkraftbedingungen im Gelkissen verbleibt. Das Gelkissen 6 ist von unten, an seiner zur Haut zeigenden Seite, mittels eines Polypropylenvlieses 10 mit einem Flächengewicht von 30 g/m² abgedeckt. Das flexible und flüssigkeitsdurchlässige Polypropylenvlies ist in seiner Größe so bemessen, dass es das Gelkissen 6 seitlich um ca. 4 bis 5 mm überragt. Dieser überstehende ringförmige Abschnitt bzw. Rand 10a ist an dem klebstoffbeschichteten Trägermaterial 1 fixiert.

Durch die dauerhafte, hautseitige Abdeckung des Gelkissens 6 mit dem dünnwandigen Vlies 10 wird verhindert, dass nach der Applikation der Elektrode auf der Haut Liquidgel in den angrenzenden Bereich der Klebstoffschicht gelangen kann.

Die hautseitigen Flächen der Elektrode werden mittels eines lösbaren Liners 8, in den durch einen Tiefziehvorgang eine Kavität 9 zur Aufnahme des überstehenden Abschnittes des Gelkissens 6 eingeformt ist, abgedeckt. Der Liner 8 deckt die klebstoffbeschichtete Seite des Trägermaterials 1 lösbar ab und bildet bis zur Applikation der Elektrode einen dauerhaften Schutz für das Gelkissen 6.

Zur elektrischen Signalübertragung sind gel- bzw. hautseitig ein mit einer Silber-Silberchlorid- Schicht überzogener Sensor 5 und an der Gegenseite ein Metalldruckknopf 4 kraftschlüssig miteinander verbunden, wobei zwischen beiden Teilen ein Label 3 eingeklemmt ist. Dieses besteht aus einer dünnwandigen Kunststofffolie, die an ihrer Oberseite bedruckt werden kann. Die Unterseite des Sensors 5 steht mit dem Gelkissen 6 in dauerhaftem Berührungskontakt. Im Betriebszustand werden die ankommenden elektrischen Signale über diese Verbindung weitergeleitet. Mittels des Labels 3, dessen Außendurchmesser deutlich größer ist als die zentrale Öffnung 2 im Trägermaterial 1, wird die Oberseite des Gelkissens 6 kraftbündig und flüssigkeitsdicht verschlossen, indem das mit Klebstoff beschichtete Label 3 an der Oberseite des Trägermaterials 1 befestigt wird. Der Liner 8 besitzt zur Ausbildung einer Grifflasche 12 eine randseitige Aussparung, die von einem streifenförmigen, klebstofffreien Abschnitt umgeben ist. Dadurch lässt sich der Liner 8 unmittelbar vor Gebrauch der Elektrode manuell leicht entfernen.

Die Liquidgelelektroden können in einem Fertigungsautomaten kostengünstig als Massenartikel hergestellt werden.

In Fig. 3 ist eine Elektrode gezeigt, die sich im Vergleich zu der Elektrode gemäß den Figuren 1 und 2 durch einen einfacheren Aufbau auszeichnet. Das Trägermaterial 1', das z.B. aus einem hautverträglichen, haftklebstoffbeschichteten Polyestervlies besteht, weist keine zentrale Öffnung auf. Das Gelkissen 6 ist direkt auf dem Trägermaterial 1' angeordnet. Der Einsatz eines Labels, wie in der ersten Ausführung, ist nicht erforderlich.

Das Gelkissen 6 (z.B. Kontur quadratisch mit Kantenlänge 18 mm mit abgerundeten Ecken, Radius 2 mm) wird nach dem Einbringen der Sensorkombination (Metalldruckknopf 4 und

Sensor 5) direkt mittig über den Sensor 5 plaziert und im Randbereich mittels einer geeigneten Vorrichtung gegen den Permanentklebstoff des Trägermaterials 1' gepresst. Anschließend wird die Abdeckung 10, die aus dem gleichen Material (Polyestervlies) wie die Abdeckung gemäß der Figuren 1 und 2 besteht (z.B. Kontur quadratisch mit Kantenlänge 23 mm mit abgerundeten Ecken Radius 3 mm), mittig über dem Sensor 5 hautseitig plaziert und im Randbereich 10a mittels einer speziellen Andrückvorrichtung auf dem haftklebstoffbeschichteten Trägervlies 1' flüssigkeitsdicht befestigt. Die anwendungstechnischen Eigenschaften dieser Ausführung sind analog wie die der zuvor beschriebenen Elektrode. Aufgrund des einfacheren Elektrodenaufbaus sind die Herstellungskosten geringer. Von Nachteil ist aufgrund des fehlenden Labels die geringere Steifigkeit im Bereich der Sensorkombination.

### Beispiel 1

Die Liquidelektroden sind analog, wie unter Bezugnahme auf die Figuren 1 und 2 beschrieben, aufgebaut und wie folgt dimensioniert:

| | |
|---|---|
| Materialdicke des Trägermaterials: | H1 = 1 mm |
| Zentrale Öffnung im Trägermaterial: | D1 = 21 mm, |
| Durchmesser Gelkissen: | D2 = 18 mm, |
| Materialdicke Gelkissen: | H2 = 3 mm |
| Durchmesser der Kavität des Liners: | D3 = 21 mm, |
| Durchmesser Abdeckung | D4 = 28 mm, |
| Höhe der Kavität (innen): | H3 = 2,2 mm |
| Menge an Liquidgel: | M1 = 0,36 g |

Die Abdeckung 10, 10a des Gelkissens 6 besteht aus einem Propylenvlies mit einem Flächengewicht von 30 g/m²

An dieser Elektrode werden folgende Eigenschaftsprüfungen durchgeführt:

### A: Bewertung der Eigenleitfähigkeit der Elektrode

Hierzu wird der Impedanzwert ACZ1 (vor Simulation der Defibrillation, ermittelt bei einer Messfrequenz von 10 Hz) zu ca. 50 Ω (Ohm) nach dem für Einwegelektroden gültigen Standard ANSI/AAMI EC12-2000 mittels Messung an Elektrodenpaaren ermittelt.

Im klinischen Gebrauch relevanter ist die Hautimpedanz, hier gemessen 3 Minuten nach Aufkleben der Elektrodenpaare auf die (unbehandelte, menschliche) Haut bei einer Meßfrequenz von 10 Hz zu ca. 100 kΩ

### B: Simulation der Fließverhältnisse des Liquidgels bei Applikation der Elektrode auf die Haut

Hierzu wird die Elektrode auf eine ebene Polyäthylenfolie aufgeklebt, indem die Elektrode vom Rand her in kreisenden Bewegungen mit einem Finger aufgedrückt wird, wie es in einschlägigen Gebrauchsinformationen zur Applikation der Elektrode auf die Haut empfohlen wird. Dieser Versuch soll zeigen, ob Gel in die Trennschicht zwischen Haftklebstoff des Trägermaterials und der Folie (als Hautersatz) gelangt oder nicht.

Wie in der praktischen Handhabung üblich und erforderlich, wird der Druckknopf 4 mittels eines dazu passenden Adapters, das zum geräteseitigen Kabelsystem gehört, an die Auswerteeinheit (Peripheriegerät) angeschlossen. Bei marktgängigen Druckknopfadaptern ist eine Aufdrückkraft von ca. 15 N erforderlich, um den mechanischen Anschluss zu erreichen. Bewertet wird der Streckenanteil auf dem Trägermaterial 1, der vom Liquidgel 7 nach einmaligem Andrückvorgang (Krafteinfluss von 20 N) auf den Druckknopf 4, ausgehend vom Außenrand der Kavität im Trägermaterial 1, in Richtung Außenkontur der Elektrode zurückgelegt wird. Je höher diese Strecke ist, um so höher ist die Wahrscheinlichkeit einer vorzeitigen Ablösung der Elektrode in der Praxis von der Haut.

### C: Praktischer Tragetest

Jeweils zwei Liquidgelelektroden werden 10 Probanden unterschiedlichen Hauttyps appliziert. Nach einer Tragedauer von 24 Stunden werden die Elektroden wieder entfernt und die Applikationsflächen werden von einer Fachkraft visuell beurteilt.

Folgende Ergebnisse wurden erhalten:
Die Eigenimpedanz nach ACZ1 betrug 60 Ω. Als Wert für die Hautimpedanz wurden 100 kΩ ermittelt.
Der Versuch B ergab, dass absolut kein Liquidgel aus dem Bereich der Kavität austritt. Nach Entfernen der Elektrode von der Folie konnte lediglich eine leichte Benetzung der Gegenfläche im Bereich des Gelkissens festgestellt werden.
Nach Abschluss des Versuches C wurden bei allen Probanden keine Veränderungen an der Haut im unmittelbaren und benachbarten Bereich der Applikationsflächen festgestellt.
Beim Entfernen der Elektroden zeigte sich, dass das Trägermaterial im gesamten, ursprünglichen Klebstoffbereich noch fest auf der Haut saß.
Auf der Haut blieb nur ein leicht feuchter Gelfilm, der nach kurzer Zeit ohne zusätzliche Reinigung von allein verschwindet.
Gemäß den vorgenommenen Untersuchungen zeichnet sich die erfindungsgemäße Liquidgelelektrode durch sehr gute Trageeigenschaften aus und gewährleistet während der Applikationsdauer eine sichere und feste Haftung auf der Haut.

### Vergleichsbeispiel 1

Es wurden Liquidgelelektroden eingesetzt, die sich von denen gemäß Beispiel 1 nur dadurch unterscheiden, dass zwischen Gelkissen 6 und Liner 8 keine Abdeckung angeordnet ist. Die Messergebnisse zur Eigenleitfähigkeit (Versuch A) waren analog wie die in Beispiel 1.

Im Versuch B zeigte sich, dass Liquidgel aus dem Gelkissen ausgetreten ist und ein Gelfilm als Trennschicht entlang einer Strecke von bis zu 10 mm deutlich zu erkennen war.

Auf der als Hautersatz dienenden PE-Folie befand sich nach Abnahme der Elektrode ein flächig-schmieriger Rückstand mit Ausläufern der Benetzung an Stellen, an denen das Gel zwischen Trägermaterial und Klebeunterlage gedrückt wurde.

Im Versuch C bestätigten sich die Ergebnisse von Versuch B.

Bei 5 von 10 Probanden löste sich bereits nach einer Tragezeit von 5 Stunden die Elektrode selbständig von der Haut. Bei drei weiteren Probanden trat diese Erscheinung nach 9 Stunden auf und bei einem weiteren Probanden nach 17 Stunden. Lediglich bei einem Probanden blieb die Elektrode während der gesamten Tragedauer auf der Haut haften. Während der Signalübertragung traten jedoch in der zweiten Hälfte der Tragedauer zeitweise Störungen auf, die ein Zeichen dafür sind, dass die Elektrode nur lose haften blieb.

Nach dem Abfallen bzw. Entfernen der Elektroden wurden die Klebeflächen visuell beurteilt.

Bei der überwiegenden Anzahl der Klebeflächen waren große Teile (über 30 % der Fläche) von einem Liquidgelfilm überzogen. Die sichere Klebwirkung war somit verschwunden bzw. nur noch schwach ausgeprägt.

An zwei Probanden wurden außerdem deutliche Hautrötungen im Bereich des hier offen liegenden Gelkissens festgestellt, die auch eine Stunde nach Entfernen der Elektroden noch nicht abgeklungen waren. Zusätzlich klagten diese Probanden über einen unangenehmen Juckreiz an dieser Stelle, insbesondere während der frühen Abendstunden. Bei einem weiteren Probanden traten die gleichen Symptome in abgeschwächter Form auf.

### Vergleichsbeispiel 2

Es wurden Liquidgelelektroden analog Beispiel 1 eingesetzt, jedoch mit dem Unterschied, dass die Einsatzmenge an Liquidgel von 0,36 g auf 0,25 g verringert wurde.

Die verringerte Gelmenge hat zur Folge, dass die Impedanz ACZ1 auf 70 Ω anstieg und die Hautimpedanz um 30%, auf 130 kΩ. Die höheren Impedanzwerte führen zu einer nicht mehr vertretbaren Beeinträchtigung der Signalqualität.

Im Versuch B zeigte sich, dass kein Gel austritt. Daher wurden auch im Versuch C ähnlich gute Ergebnisse wie in Beispiel 1 erzielt.

Aufgrund der schlechten Signalübertragung ist diese Elektrode jedoch für einen praktischen Einsatz ungeeignet.

### Bezugszeichen

- 1: Trägermaterial
- 1': Trägermaterial
- 2: Öffnung
- 3: Label
- 4: Druckknopf
- 5: Sensor
- 6: Gelkissen
- 7: Liquidgel
- 8: Liner
- 9: Kavität
- 10: Abdeckung
- 10a: umlaufender Rand
- 11: Klebstoffbeschichtung
- 12: Grifflasche

## Patentansprüche

1. Biomedizinische, nichtinvasive, selbstklebende, flexible Elektrode zum einmaligen Gebrauch für die Zu- oder Ableitung elektrischer Signale über die Haut von Mensch oder Tier, bestehend aus einem Trägermaterial (1, 1'), einem Gelkissen (6), das mit einem ionisch leitfähigen Liquidgel (7) getränkt ist, einem an der zur Haut zeigenden Seite angeordneten ablösbaren Liner (8), einem elektrisch leitenden Sensor (4, 5), der mit dem Gelkissen (6) in Kontakt steht und über eine elektrische Anschlussleitung mit einem peripheren Gerät verbunden ist, **dadurch gekennzeichnet, dass** zwischen dem ablösbaren Liner (8) und der zur Haut zeigenden Seite des Gelkissens (6) eine dünnwandige, flexible, flüssigkeitsdurchlässige, nassfeste Abdeckung (10, 10a) angeordnet ist, die so dimensioniert ist, dass diese die in Richtung zur Haut zeigende Fläche des Gelkissens (6) vollständig überdeckt und einen umlaufenden Befestigungsrand (10a) aufweist, der an zur Haut zeigenden Seite des Trägermaterials (1, 1') dauerhaft fest mit diesem verbunden ist, und die zur Haut zeigende Fläche der Abdeckung (10) während der Applikation der Elektrode mit der Haut in Berührungskontakt steht, wobei Liquidgel (7) durch die Abdeckung (10) hindurch an den vom Gelkissen (6) überdeckten Bereich der Hautoberfläche gelangt.

2. Elektrode nach Anspruch 1, **dadurch gekennzeichnet, dass** die Abdeckung (10) aus einem vliesartigen Material mit einem Flächengewicht von 5 bis 80 g/cm², vorzugsweise 15 bis 30 g/cm² besteht.

3. Elektrode nach Anspruch 1, **dadurch gekennzeichnet, dass** die Abdeckung (10) aus einem folienartigen, perforierten Polymermaterial mit einer Dicke von 0,01 bis 0,25 mm besteht.

4. Elektrode nach einem der Ansprüche 1 bis 3, **dadurch gekennzeichnet, dass** die Fläche der Abdeckung (10), die mit der Haut in Kontakt gelangt, eine samtweiche Oberfläche besitzt.

5. Elektrode nach einem der Ansprüche 1 bis 4, **dadurch gekennzeichnet, dass** das Trägermaterial (1) eine Öffnung (2) besitzt, in die das Gelkissen (6) eingesetzt ist, der Rand (10a) der Abdeckung (10) für das Gelkissen (6) an dem Trägermaterial (1) mittels einer Klebstoffschicht flüssigkeitsdicht befestigt ist, auf der hautabgewandten Seite ein Label (3) angeordnet ist, das das Gelkissen (6) vollständig überdeckt und flüssigkeitsdicht mit dem Trägermaterial (1) verbunden ist und die zur Haut zeigende Seite des Trägermaterials vollflächig mit einem ablösbaren Liner (8) verbunden ist, der eine Kavität besitzt, die den überstehenden Teil des Gelkissens (6) aufnimmt.

6. Elektrode nach einem der Ansprüche 1 bis 4, **dadurch gekennzeichnet, dass** das Gelkissen (6) auf der zur Haut zeigenden Seite eines flüssigkeitsdichten, vollflächigen Trägermaterials (1') angeordnet ist, der Rand (10a) der Abdeckung (10) für das Gelkissen (6) an dem Trägermaterial (1') mittels einer Klebstoffschicht flüssigkeitsdicht befestigt ist und die zur Haut zeigende Seite des Trägermaterials (1') vollflächig mit einem ablösbaren Liner (8) verbunden ist, der eine Kavität besitzt, die den überstehenden Teil des Gelkissens (6) aufnimmt.

7. Elektrode nach einem der Ansprüche 1 bis 6, **dadurch gekennzeichnet, dass** der Sensor (4, 5) entweder in dem Label (3) oder in dem Trägermaterial (1') fixiert ist.

8. Elektrode nach einem der Ansprüche 1 bis 7, **dadurch gekennzeichnet, dass** der Rand (10a) der Abdeckung (10) mittels eines unter Druck und Wärme schmelzbaren Klebstoffes befestigt ist.

9. Elektrode nach einem der Ansprüche 1 bis 8, **dadurch gekennzeichnet, dass** die Tränkung des Gelkissens (6) mit Liquidgel erst unmittelbar vor dem Aufsetzen des Liners (8) erfolgt.
